# EUROPEAN PATENT SPECIFICATION

(11) **EP 3 088 862 B1**
(45) Date of publication and mention of the grant of the patent: **10.06.2020**
(21) Application number: 16165446.2
(22) Date of filing: 14.04.2016
(51) Int. Cl.: G01N 7/00, G01F 22/02, G01F 15/08, G01F 23/72, G01F 15/07, G01F 1/74

(54) **APPARATUS AND METHOD FOR DETERMINING AN AMOUNT OF NON-CONDENSABLE GAS**
VORRICHTUNG UND VERFAHREN ZUR BESTIMMUNG EINES NICHTKONDENSIERBAREN GASES
APPAREIL ET PROCÉDÉ PERMETTANT DE DÉTERMINER UNE QUANTITÉ DE GAZ NON CONDENSABLE

(30) Priority: 30.04.2015 GB 201507439
(43) Date of publication of application: 02.11.2016
(73) Proprietor: Spirax-Sarco Limited, Cheltenham, Gloucestershire GL53 8ER (GB)
(72) Inventor: JOHNSON, Nick, Cheltenham, Gloucestershire GL51 9NQ (GB); HUNT, Gareth, Cheltenham, Gloucestershire GL51 9NQ (GB); MCKENNA, Paul, Cheltenham, Gloucestershire GL51 9NQ (GB); CHANNON, Paul, Cheltenham, Gloucestershire GL51 9NQ (GB)
(74) Representative: Haseltine Lake Kempner LLP

(56) References cited:
- EP-A1- 1 020 713
- AU-A- 6 323 373
- DE-A1- 2 846 826
- DE-A1- 19 714 168
- DE-U1-202013 004 024
- FR-A1- 2 841 963
- GB-A- 2 089 049
- US-A- 3 967 494
- US-A1- 2005 115 314
- US-A1- 2014 238 124

## Description

The invention relates to an apparatus and method for determining an amount of non-condensable gas in a fluid flow comprising liquid condensate and non-condensable gas.

The presence of non-condensable gas, such as air, within a steam flow can significantly alter the properties of the steam flow. For example, non-condensable gas may affect the heat transfer properties of the steam flow. Further, non-condensable gas may lead to heterogeneous steam properties within the steam flow, such as regions of superheated steam and regions of saturated steam.

In a number of industrial processes, it is desirable to minimise the amount of non-condensable gas within a steam flow. One such example is the sterilisation of products and/or equipment in a steam flow, for example in the medical and pharmaceutical industries. If the amount of non-condensable gas within the steam flow is too high, the heat transfer rate between the steam flow and the equipment may be insufficient to properly sterilise the equipment.

It is known to estimate the amount of non-condensable gas in a steam flow by condensing a sample of the steam and collecting the resultant non-condensable gas and condensate. European Standard EN 285 defines a safety limit of 3.5 ml of non-condensable gas per 100ml of condensate, or 3.5%, for sterilisation applications.

Non-condensable gas may be introduced into a steam system in a number of ways. For example, non-condensable gas can be introduced by excessive aeration during water treatment, such as water softening. Further, non-condensable gas can be introduced into a steam system upstream of a boiler if the inlet water for the boiler is not pre-heated sufficiently, since non-condensable gas (e.g. air) is more easily absorbed in water at lower temperatures. Insufficient pre-heating may occur in a variable demand steam system when the amount of steam demanded from the boiler is increased, therefore increasing the flow rate of inlet water through the pre-heater to the boiler.

Traditional methods of evaluating the amount of non-condensable gas in a fluid flow involve the extraction of a portion of the flow, condensing the flow to provide a mixture of liquid and non-condensable gas, and the introduction of the extracted condensed mixture into an upturned vessel submerged in a liquid-filled container. A non-condensable gas portion of the fluid flow collects in the upturned vessel, whereas the liquid is displaced from the container by both the liquid portion of the fluid flow and the non-condensable gas portion. The proportion of non-condensable gas can be estimated by measuring the volume of gas and displaced liquid respectively.

However, such manual methods are time-consuming and may be inaccurate.

Automatic methods have previously been considered which rely on diverting the extracted and condensed flow mixture through a monitoring tube sized so that the flow consists of alternating sections of condensed liquid and non-condensable gas. The amount of non-condensable gas in the flow mixture can be estimated by monitoring the phase and time length of each section passing a sensor. However, such methods may rely on expensive equipment to accurately assess the time length of each section, or indeed the phase.

Other methods have previously been considered for automatic monitoring, but typically rely on sensors for determining the volume of non-condensable gas, once separated from the condensate. Owing to the typically small quantities of non-condensable gas, particularly in regulated applications of steam flows, the gas sensors are required to be very sensitive and therefore are typically expensive.

Accordingly, it is desirable to provide an alternative and/or improved method of determining the amount of non-condensable gas in a fluid flow.

DE 20 2013 004024 discloses an apparatus for determining a non-condensable gas (NCG) content in a sterilization steam, the sterilization steam being in the form of an NCG condensate mixture. The apparatus comprises a gas collecting unit having an inner tube, an outer tube, and an inflow opening for introducing the NCG condensate mixture. The inflow opening has a first control element for opening or closing the inflow opening, and an overhead outflow opening, for discharging the NCG, has a second control element for opening or closing the outflow opening. The outer pipe has an overflow for the condensate arranged between the inflow opening and the outflow opening. The inner tube has a first level detection means, and comprises a condensate collecting unit having a discharge opening for discharging condensate. The condensate collection unit has a second level detection means. The apparatus comprises a control and evaluation unit which is connected to the first and second level detection means which calculates the amount of NCG in the gas collecting unit and the amount of condensate in the condensate collecting unit to determine the amount of the NCG in the mixture.

According to an aspect of the invention there is provided a method of determining the amount of non-condensable gas within a fluid flow containing both non-condensable gas and condensate liquid, the method being specified by appended independent claim 1.

Determining the amount of non-condensable gas may comprise determining a non-condensable gas parameter relating to the amount of non-condensable gas in the fluid flow. For example, the non-condensable gas parameter may be a volumetric proportion of non-condensable gas in the fluid flow, or may be a volumetric proportion of non-condensable gas adjusted to reflect the volume of non-condensable gas in standardized or alternative pressure and temperature conditions.

The level signal may relate to the height of the liquid level in the fluid displacement volume and/or the rate of change of the height of the liquid level in the fluid displacement volume. The liquid flow signal may relate to the volume or mass of liquid discharged from the fluid displacement volume and/or the volumetric or mass flow rate of liquid discharged from the liquid displacement volume.

The liquid level may be monitored using a level sensor comprising a float disposed in the fluid displacement volume and a position sensor for monitoring the position of the float and generating the level signal relating to the liquid level in the fluid displacement volume. The discharge of liquid from the fluid displacement volume may be monitored by a flow meter disposed downstream of the fluid displacement volume. The flow meter may be a flow totalizer. The flow meter may be a target-type flow meter in which a target is embedded in the liquid flow.

The level sensor may comprise a magnetostrictive sensor configured to detect movement of the moveable float relative to a static part.

The method may further comprise switching from a refill configuration to a discharge configuration when the liquid level reaches a first predetermined level. In the refill configuration a liquid outlet port may be closed and a gas outlet port may be configured to discharge gas from the fluid displacement volume; and in the discharge configuration the liquid outlet port may be configured to discharge liquid from the fluid displacement volume and the gas outlet port may be closed.

The method may further comprise switching from the discharge configuration to the refill configuration when the liquid level reaches a second predetermined level lower than the first predetermined level. In the discharge configuration the liquid level in the fluid displacement volume may reduce from the first liquid level towards the second liquid level as the fluid flow is received in the fluid displacement volume.

The flow of liquid from the fluid displacement volume may be controlled using a pressure maintaining valve configured to maintain a pre-determined back pressure. For example, the pre-determined back pressure may be 0.5 bar gauge. The pressure maintaining valve may be disposed downstream of the fluid displacement volume. The discharge of liquid from the displacement volume may be monitored downstream of the fluid displacement volume and upstream of the pressure maintaining valve, or downstream of both the fluid displacement volume and the pressure maintaining valve. Maintaining a pre-determined back pressure may enable the calculation of the quantity of non-condensable gas to be substantially unaffected by changes in ambient pressure or drain pressure.

The flow of gas from the fluid displacement volume may be controlled using a pressure maintaining valve configured to maintain a pre-determined back-pressure. The pressure maintaining valve may be disposed downstream of or integral with a three-port valve configured to switch between the discharge and refill configurations. In other words, the pressure maintaining valve may be arranged in series with a three-port valve, or the pressure maintaining valve may include a three-port valve. Accordingly, the same valve may control both liquid and gas flows, and so substantially the same back pressure may be maintained in the fluid displacement volume irrespective of whether gas (through a gas outlet port) or liquid (through a liquid outlet port) is being discharged. The three port valve may also be referred to as a solenoid valve or switchover valve.

Alternatively, the gas outlet port may have a gas outlet valve independent of a pressure maintaining valve for the liquid outlet port, and the back pressure of the pressure maintaining valve for the liquid outlet port may correspond to operation in the refill configuration with the liquid level at the first predetermined liquid level. The gas outlet port may be opened and closed by opening and closing the gas outlet valve accordingly. In other words, the first liquid level may be predetermined by virtue of the threshold pressure at the pressure maintaining valve at which it is set to discharge liquid.

The first liquid level may be at least 50%, at least 60%, at least 70%, at least 80%, at least 90%, at least 95% or substantially 100% of the height of the fluid displacement volume.

The liquid level in the fluid displacement volume may reduce from the first liquid level towards the second liquid level as the fluid flow is received in the fluid displacement volume with the gas outlet port closed. Accordingly, liquid may be discharged from the displacement volume as fluid flow is received and the liquid level reduces. The volume and rate of liquid discharged from the displacement volume are therefore equivalent to the volume and rate of fluid flow entering the fluid displacement volume whilst the gas outlet port is closed.

The gas outlet port may be opened when it is determined that the liquid level in the fluid displacement volume reaches the second liquid level, and may remain open until the liquid level in the fluid displacement volume subsequently returns to the first liquid level.

The amount of non-condensable gas is determined for a known geometry of the fluid displacement volume and may more specificly be determined by: determining a gas flow parameter relating to a gas volume or gas flow rate into the fluid displacement volume over a time period based on the level signal; determining a total flow parameter relating to the combined liquid and gas volume or combined liquid and gas flow rate into the fluid displacement volume over the time period based on the liquid flow signal; and determining a non-condensable gas parameter based on the based on the gas flow parameter and the total flow parameter.

The time period may be pre-determined or may be set based on a volume parameter relating to the gas volume (i.e. as derived from the level signal) or the combined liquid and gas volume (i.e. as derived from the liquid flow signal). For example, the time period may be set so that a monitoring period corresponds to a minimum gas volume accumulated in the fluid displacement volume.

The amount of non-condensable gas may be determined for a monitoring period corresponding to a reduction in the liquid level that is less than the separation between the first and second pre-determined liquid levels. Accordingly, the amount of non-condensable gas may be determined more frequently than the period over which the liquid level reduces from the first pre-determined level to the second pre-determined level in use. The non-condensable gas parameter may be determined based on a rolling average calculation as the liquid level reduces.

The amount of non-condensable gas may represent the amount of non-condensable gas as a proportion of the fluid flow (i.e. the total flow) or as compared to the liquid component of the fluid flow.

The method may further comprise monitoring the temperature in the fluid displacement volume, and the amount of non-condensable gas may be determined based on the liquid level signal, the liquid flow signal and the temperature in the fluid displacement volume. Accordingly, the amount of non-condensable gas may be determined taking into account the compressibility of the non-condensable gas in the fluid displacement volume.

The amount of non-condensable gas may be determined based at least partly on the pressure of the fluid in the fluid displacement volume. The pressure of the fluid in the displacement volume may be determined by virtue of a predetermined relationship between the pressure in the fluid displacement volume and a pre-determined pressure setting of the pressure maintaining valve for discharging liquid (i.e. the back pressure maintained at the valve). Alternatively, the pressure may be monitored using a pressure sensor for monitoring the pressure of gas and/or liquid within the fluid displacement volume. For example, a pressure sensor may be disposed at an upper portion of the fluid displacement volume (i.e. at the upper end) so as to monitor the pressure of non-condensable gas that has risen within the volume.

The method may further comprise condensing an upstream flow comprising non-condensable gas and condensable gas to provide the fluid flow comprising condensate liquid and non-condensable gas.

The fluid displacement volume may comprise a plurality of interconnected fluid displacement chambers, and the fluid flow may be received in a first fluid displacement chamber, and the level sensor may monitor the liquid level of a free surface in a second fluid displacement chamber to generate the level signal. The cross-sectional area of the first fluid displacement chamber may be less than the cross-sectional area of the second fluid displacement chamber.

The fluid flow may be received in the fluid displacement volume along a generally upward direction. Accordingly, the gas bubbles within the fluid flow may be directed generally upwardly so that entrainment of the gas bubbles in the liquid component of the flow is reduced.

Alternatively, the fluid flow may be received in the fluid displacement volume along a substantially horizontal or at least partly lateral direction so that the fluid flow tends to swirl in the fluid displacement volume. The swirl may tend to increase the distance travelled by the fluid flow, thereby increasing the opportunity for gas bubbles to separate from the liquid portion of the fluid flow and migrate upwardly, as opposed to being entrained with the liquid portion of the fluid flow.

According to a second aspect of the invention there is provided an apparatus for determining the amount of non-condensable gas within a fluid flow containing both non-condensable gas and condensate liquid, the apparatus being specified by appended independent claim 12.

The non-condensable gas determining module may be configured to determine a non-condensable gas parameter relating to the amount of non-condensable gas in the fluid flow. For example, the non-condensable gas parameter may be a volumetric proportion of non-condensable gas in the fluid flow, or may be a volumetric proportion of non-condensable gas adjusted to reflect the volume of the non-condensable gas under standardized or alternative pressure and/or temperature conditions.

The level sensor may comprise a float disposed in the fluid displacement volume and moveable in response to changes in the liquid level, and a position sensor for monitoring the position of the float and generating the level signal. The flow meter may be disposed downstream of the fluid displacement volume. The meter may be a flow totalizer.

The apparatus may further comprise a gas outlet port for exhausting gas from the fluid displacement volume. The apparatus may further comprise a liquid outlet port for controlling the discharge of liquid from the fluid displacement volume.

The apparatus may be configured to switch from a refill configuration to a discharge configuration when the liquid level reaches a first predetermined level. In the refill configuration the liquid outlet port may be closed and the gas outlet port may be configured to discharge gas from the fluid displacement volume. In the discharge configuration the liquid outlet port may be configured to discharge liquid from the fluid displacement volume and the gas outlet port may be closed.

The apparatus may be configured to switch from the discharge configuration to the refill configuration when the liquid level reaches a second predetermined level lower than the first predetermined level. The apparatus may comprise a controller configured to switch between the discharge and refill configurations.

The apparatus may further comprise a pressure maintaining valve configured to maintain a pre-determined back pressure, and the pressure maintaining valve may be configured to control liquid and/or gas discharge through the liquid outlet port and/or the outlet port respectively.

The pressure maintaining valve may comprise a three-port valve configured to switch between the discharge and refill configurations. In other words, the three-port valve may control flow through both the gas outlet port and the liquid outlet port, and may switch between the discharge and refill configurations of the apparatus accordingly.

The pressure maintaining valve may comprise a three-port valve (i.e. the three-port valve may be integral with the pressure maintaining valve), or alternatively the pressure maintaining valve may be disposed downstream of a separate three-port valve.

The non-condensable gas determining module is configured to determine the amount of non-condensable gas for a known geometry of the fluid displacement volume and may more specificly be configured to determine the amount of non-condensable gas by: determining a gas flow parameter relating to a gas volume or gas flow rate into the fluid displacement volume over a time period based on the level signal; determining a total flow parameter relating to the combined liquid and gas volume or combined liquid and gas flow rate into the fluid displacement volume over the time period based on the liquid flow signal; and determining a non-condensable gas parameter based on the gas flow parameter and the total flow parameter.

The apparatus may further comprise a temperature sensor configured to generate a temperature signal relating to the temperature of non-condensable gas in the fluid displacement volume. The non-condensable gas determining module may be configured to determine the amount of non-condensable gas based on the liquid level signal, the liquid flow signal and the temperature signal.

The apparatus may be configured so that the liquid flow is received along a substantially horizontal or at least partly upward direction.

The invention will now be described, by way of example, with reference to the attached drawings, in which:
Figure 1 schematically shows apparatus for determining an amount of non-condensable gas with a float of the level sensor in a first position corresponding to a maximum liquid level;
Figure 2 schematically shows the apparatus of Figure 1 with the float in a second position corresponding to the minimum liquid level.

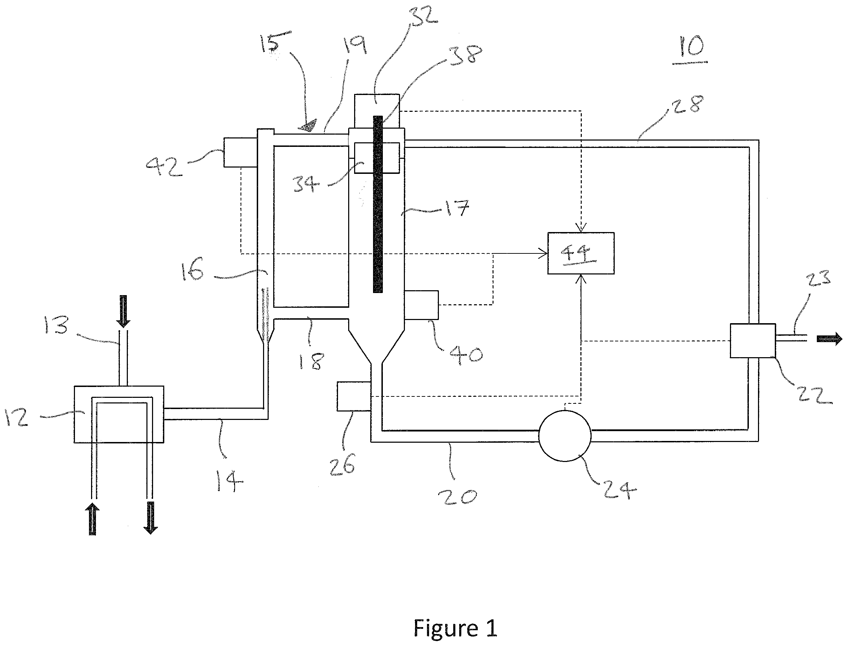

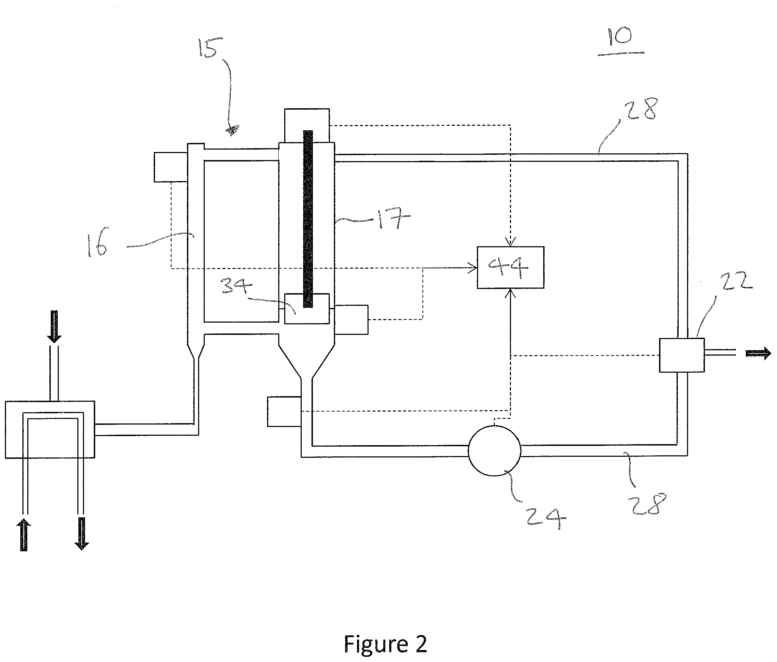

As shown in **Figures 1** **and** **2****,** an apparatus 10 for determining an amount of non-condensable gas comprises a condenser 12 having an inlet 13 for receiving a fluid flow such as a steam sample from a steam system, and a delivery line 14 for discharging a condensed fluid flow to a fluid displacement volume 15 of the apparatus.

The fluid displacement volume 15 comprises first and second substantially vertically oriented and interconnected fluid displacement chambers 16, 17, in the form of parallel cylindrical columns. In this embodiment, the first displacement chamber 16 has a diameter of approximately 11mm whereas the second displacement chamber 17 has a diameter of approximately 30mm. Each displacement chamber 16, 17 has a cylindrical section having a length of approximately 110mm.

The fluid displacement volume 15 is fluidically coupled to the delivery line 14 so that in use the condensed fluid flow from the condenser 12 enters the lower end of the first fluid displacement chamber 16. In this embodiment the delivery line 14 is coupled to the first fluid displacement chamber 16 so that the fluid flow is delivered into the chamber 16 along a substantially vertical direction with respect to the axis of the chamber 16. Accordingly, the gas bubbles in the fluid flow may tend to rise owing to their momentum on entry to the volume 15, and may avoid becoming entrained with the liquid portion of the flow.

The first and second fluid displacement chambers 16, 17 are parallel and have substantially the same axial extent. The two chambers 16, 17 are connected by lower and upper channels 18, 19 extending between lower and upper ends of the chambers 16, 17 respectively to fluidically couple the chambers.

The delivery line 14 extends into the first displacement chamber 16 so that a nozzle at the end of the delivery 14 is positioned above the lower channel 18 interconnecting the two chambers 16,17. Further, the nozzle is provided with a divergent cross-section so as to encourage the liquid component of the flow to turn around the lip of the nozzle and downwardly towards the interconnecting channel 18, as described below. Extending the delivery line 14 into the first displacement chamber 16 as described above prevents gas bubbles within the fluid flow from flowing laterally through the lower interconnecting channel 18, thereby avoiding discharge of the gas bubbles along the liquid outlet line 20 (described below).

The second fluid displacement chamber 17 has a downwardly tapering frustoconical wall at its lower end leading to a liquid outlet line 20 for discharging liquid condensate from the fluid displacement volume 15. The liquid outlet line 20 extends to a valve 22, which is a pressure maintaining valve (otherwise known as a surplussing valve) configured to automatically open when at least a threshold pressure is experienced at the valve 22, and close when a pressure less than the threshold is experienced at the valve.

A flow meter 24, which in this embodiment is a flow totalizer, is installed in the liquid outlet line 20 for monitoring the flow rate and volume (over discretised time periods) between the fluid displacement volume 15 and the liquid outlet valve 22. In other embodiments, the flow meter 24 may be installed downstream of the liquid outlet valve 22.

There is also a temperature sensor 26 arranged to monitor the temperature of the liquid in the liquid outlet pipe 20 and generate a liquid outlet temperature signal accordingly.

Towards the upper end of the fluid displacement volume 15 (e.g. in the upper fifth of the volume), there is a gas outlet fluidically coupled to a gas outlet line 28 for exhausting gas (in particular non-condensable gas) from the fluid displacement chamber 15. In this embodiment, the gas outlet line 28 opens into the second fluid displacement chamber 17.

The gas outlet line 28 also extends to the pressure maintaining valve 22, which in this embodiment is a three-port valve (or solenoid or switchover valve). The three-port valve 22 has a discharge configuration in which the liquid outlet line 20 is in communication with a discharge line 23 so that a liquid outlet port of the line 20 is configured to discharge liquid (when the liquid is above the threshold back pressure) and so that a gas outlet port of the gas outlet line 28 is closed. The three-port valve 22 also has a refill configuration in which the gas outlet line 28 is in communication with the discharge line 23, so that the gas outlet port is configured to discharge gas (when the gas is above the threshold back pressure) and so that the liquid outlet port of the liquid outlet line is closed.

The apparatus 10 further comprises a level sensor 32 comprising a float 34 configured to float on a free-surface of condensate (i.e. the surface between liquid condensate and non-condensable gas) within the second fluid displacement chamber 17 and coupled to a position sensor 36 by a static rod 38. The position sensor 36 is configured to generate a signal corresponding to the position of the float in the fluid displacement chamber, and therefore monitors the liquid level of the free surface in the displacement chamber 18. In this embodiment, the level sensor 32 is a magnetostrictive sensor, and the position sensor 36 is configured to generate a signal in response to movement of the float along the rod. Since the float 34 is disposed in the second fluid displacement chamber 17, it is not directly affected by any rising bubbles of non-condensable gas within the first displacement chamber 16. In contrast, if the float 34 were disposed in the first displacement chamber, the position of the float may be affected by pressure or buoyancy effects of the bubbles rising and impacting the underside of the float 34.

The fluid displacement volume 15 is also provided with pressure and temperature sensors 40, 42 for monitoring the pressure and temperature in the fluid displacement chamber 18. The temperature sensor 42 is located towards the upper end of the fluid displacement volume 15 (i.e. above the first liquid level, as will be described below) so as to directly monitor the temperature of non-condensable gas that collects therein.

The pressure sensor 40 is provided in this embodiment for confirming that the pressure in the fluid displacement volume 15 is maintained at a constant level by the pressure maintaining valve 22. The pressure sensor 40 is therefore provided towards the lower end of the fluid displacement volume 15 (i.e. below the second liquid level, as will be described below), where the pressure level of the liquid in the fluid displacement volume 15 should be constant in use, irrespective of the level or height of the free surface in the fluid displacement chamber.

The pressure sensor 40 is provided so that compressibility effects on the non-condensable gas collecting in the upper end of the volume 15 may be accounted for.

The apparatus 10 also comprises a controller 44 for controlling the operation of the apparatus 10 and a non-condensable gas determining module (which in this embodiment is integral with the controller 44) for determining the amount of non-condensable gas in the fluid flow.

The controller 44 is configured to control the apparatus to conduct the monitoring method described in detail below.

In this example, a steam sample flow is diverted from a steam system (not shown), such as a steam sterilisation system, to the condenser 12. The condenser 12 causes the steam sample flow to be condensed to provide a fluid flow comprising both non-condensable gas and condensate liquid, which flows continuously into the fluid displacement chamber 15 via the delivery line 14.

In a startup and/or recalibration phase of the method, the controller 44 causes the pressure maintaining valve to switch to the refill configuration so that gas may be discharged from the fluid displacement volume 15, thereby allowing the fluid displacement volume 15 to fill to a first liquid level. In this embodiment, the first liquid level is set by the controller 44 as a position corresponding to 80% of the height of the fluid displacement volume 15. When it is determined by the level sensor 32 that the liquid level has reached the first liquid level, the controller 44 causes the pressure maintaining valve 22 to switch from the refill configuration to the discharge configuration. Since both the gas outlet line 28 and the liquid outlet line 20 are connected to the same pressure maintaining valve 22, the pressure remains substantially constant in the fluid displacement volume 15 when the valve switches over.

Accordingly, as further fluid flow is received in the fluid displacement volume 15, the non-condensable gas accumulates in the upper portions of the fluid displacement chambers 16, 17 and liquid is caused to flow through the pressure maintaining valve 22.

As non-condensable gas received in the fluid displacement volume 15 rises and accumulates in the upper portion of the volume 15, liquid is displaced and the liquid level is caused to reduce. The float 34 moves downwardly with the liquid level, and the position sensor 36 generates a level signal representative of the liquid level accordingly, which is communicated to the controller 44.

Since the gas outlet port of the gas outlet line 28 is closed in the discharge configuration, liquid is discharged from the fluid displacement chamber at the same rate as the rate of the fluid flow entering the fluid displacement volume 15 (i.e. the fluid flow comprising both non-condensable gas and liquid). The flow meter 24 in the liquid outlet line 20 monitors the flow of liquid from the fluid displacement volume, and generates a liquid flow signal accordingly, which in this embodiment relates to the volume of liquid discharged over discretised time periods. The liquid flow rate, or an output proportional to the liquid flow rate, may be determined by obtaining the derivate of the liquid flow signal. In other embodiments, the liquid flow signal may represent the liquid flow rate (or may be proportional to the liquid flow rate), and an output equivalent to (or proportional to) the volume discharged over a time period may be obtained by way of integrating the signal.

The controller 44 determines the amount of non-condensable gas using the level signal and the liquid flow signal at regular time intervals, for example at intervals of 20 seconds.

The controller 44 calculates the volume of non-condensable gas accumulated in the time period based on the change in the level of the free-surface in the fluid displacement volume, and the known geometry (i.e. cross-sectional area) of the fluid displacement volume 15. Further, the controller 44 calculates the volume of fluid flow received based on the volume of liquid flow through the flow meter 24 in the same time period.

In this embodiment, the controller 44 calculates the corresponding volume of the non-condensable gas under the temperature and pressure conditions specified under European Standard EN 285, using the ideal gas law. This is done based on the known pressure and temperature of the non-condensable gas in the fluid displacement chamber, as calculated based on the back-pressure maintained by the valve 22 and/or the pressure monitored by the pressure sensor 40, and by the temperature sensor 42 respectively; together with the pressure and temperature associated with the standard. The pressure sensors are calibrated using a further barometric pressure sensor (not shown). Similarly, the controller 44 calculates the corresponding volume of the condensate under the conditions in EN 285 based on the known temperature and pressure of the condensate in the fluid displacement chamber 18 and liquid outlet line 20, and the corresponding pressure and temperature in the standard.

The controller 44 therefore calculates a non-condensable gas parameter in the form of the volumetric ratio of non-condensable gas to condensate as required by EN 285. If this ratio is found to be greater than 3.5%, the controller generates an alarm.

The controller 44 stores the non-condensable gas parameter calculated for each time period in a memory.

As non-condensable gas continues to accumulate in the fluid displacement chamber, the liquid level will drop to a second predetermined liquid level (as shown in Figure 2), which in this embodiment corresponds to 20% of the height of the cylindrical portion of the fluid displacement chambers 16, 17. The controller 44 determines that the second predetermined liquid level has been reached based on the level signal, and causes the pressure maintaining valve 22 to switch to the refill configuration. Accumulated non-condensable gas is thereby discharged from the fluid displacement chamber through the gas outlet line 28. Consequently, the liquid outlet valve 22 ceases allowing liquid to flow therethrough and the liquid level in the fluid displacement chamber rises towards the first liquid level as fluid flow continues to be received therein.

When the controller 44 determines, based on the level signal, that the liquid level has returned to the first liquid level, the controller 44 causes the pressure maintaining valve 22 to return to the discharge configuration, thereby ending a monitoring cycle. The monitoring cycle is repeated whilst the apparatus remains in use.

At the end of each monitoring cycle, the controller 44 determines a cycle-based non-condensable gas parameter (i.e. the amount of non-condensable gas) for the time period associated with the cycle (i.e. corresponding to the movement of the liquid level from the first liquid level to the second liquid level). This calculation is performed in the same way as the regular time-interval calculation described above, but based on the total volume of non-condensable gas accumulated as the liquid level moves from the first liquid level to the second liquid level, and the total volume of liquid that flowed through the flow meter 24 in the same time period.

The controller 44 records the start and end of each cycle, in order to calculate the volume of at least the liquid flow through the flow meter 24 during the time period of the cycle based on the recorded liquid flow signal. In other embodiments, the flow meter may record a volumetric flow count for the liquid flow signal, which may be initialised at the start of each monitoring cycle and stopped at the end. Further, the controller 44 may determine the volume of non-condensable gas based on the known geometry of the fluid displacement chamber and the known distance between the first and second liquid levels, without reference to the profile of the liquid level signal over the time period of the monitoring cycle.

In embodiments, the controller 44 stores a time-correlated log of the positions of the float for subsequent analysis.

It will be appreciated that the accuracy of the level sensor improves as the liquid level reduces from the first liquid level, as any inherent error affecting the position sensor becomes increasingly negligible as the distance between the current liquid level and the first liquid level increases. Accordingly, the accuracy of the level sensor may be greatest at the end of the monitoring cycle when the level of the free surface has reduced from the first liquid level to the second liquid level. Correspondingly, it will also be appreciated that the accuracy of the level sensor improves dependent on the travel between the first and second liquid levels, which depends on the geometry of the fluid displacement volume. Consequently, tall and thin fluid displacement chambers may result in improved accuracy when compared with squat fluid displacement chambers.

The cycle-based non-condensable gas parameter may be stored or used differently from the non-condensable gas parameter calculated at prescribed or relatively short regular time intervals. For example, the non-condensable gas parameter calculated at regular time intervals may be displayed on a live display (such as a monitor), and/or used for an alarm configured to determine if the amount of non-condensable gas exceeds a threshold. The non-condensable parameter as calculated at regular time intervals may therefore be used for monitoring the variability of the amount of non-condensable gas in the fluid flow, and detecting peaks of non-condensable gas. This parameter may or may not be stored in long-term memory. The cycle-based non-condensable gas parameter will typically be based on a longer time period of monitoring, and is therefore more representative of typical levels of non-condensable gas as averaged over this longer duration. For example, the time period for each monitoring cycle may be between 5 minutes and 30 hours. The record of the cycle-based non-condensable gas parameter may be stored in long term memory.

The apparatus of the invention enables the amount of non-condensable gas in a fluid flow to be monitored simply and inexpensively, since relatively inexpensive sensors such as a position sensor can be used for determining the liquid level of the free surface in the fluid displacement chamber, and a flow totalizer or flow meter can be used for monitoring the liquid flow from the displacement chamber.

In other embodiments, a non-condensable gas parameter may additionally or alternatively be calculated based on the level signal and liquid flow signal on demand for a given monitoring period. Further, the non-condensable gas parameter may be calculated at intervals corresponding to predetermined volumes of non-condensable gas (as monitored by the level sensor) or fluid flow (as monitored by the flow meter), for example 10ml of non-condensable gas or 1000ml of fluid flow.

Although an embodiment is shown in which the pressure maintaining valve is a three-port valve configured to switch between receiving fluid from the gas outlet line and the liquid outlet line and discharging the fluid to the drain, it will be appreciated that in other embodiments there may be a separate three-port valve upstream of the pressure-maintaining valve. Alternatively, each outlet line may have a respective pressure maintaining valve, which may be controlled to switch between the refill and discharge configurations of the apparatus.

## Claims

1. A method of determining the amount of non-condensable gas within a fluid flow containing both non-condensable gas and condensate liquid, the method comprising:
receiving the fluid flow in a fluid displacement volume (15) causing the non-condensable gas to rise and accumulate in an upper portion of the volume (15); and
monitoring the liquid level of a free surface in the fluid displacement volume (15) to generate a level signal; **characterised in**
monitoring the flow of liquid out of the fluid displacement volume (15) while the liquid is discharged from the fluid displacement volume at the same rate as the rate of the fluid flow entering the fluid displacement volume and a gas outlet line of the fluid displacement volume is closed, to generate a liquid flow signal related to the flow rate of liquid out of the fluid displacement volume (15); and
determining the amount of non-condensable gas in the fluid flow based on the level signal and the liquid flow signal, wherein the determination is for a known geometry of the fluid displacement volume.

2. A method according to claim 1, wherein the liquid level is monitored using a level sensor (32) comprising a float (34) disposed in the fluid displacement volume (15) and a position sensor (36) for monitoring the position of the float (34) and generating the level signal relating to the liquid level in the fluid displacement volume (15).

3. A method according to any preceding claim, wherein the flow of liquid out of the fluid displacement volume (15) is monitored by a flow meter (24) disposed downstream of the fluid displacement volume (15).

4. A method according to any preceding claim, further comprising switching from a refill configuration to a discharge configuration when the liquid level reaches a first predetermined level; wherein in the refill configuration a liquid outlet port is closed and a gas outlet port is configured to discharge gas from the fluid displacement volume (15); and wherein in the discharge configuration the liquid outlet port is configured to discharge liquid from the fluid displacement volume (15) and the gas outlet port is closed.

5. A method according to claim 4, further comprising switching from the discharge configuration to the refill configuration when the liquid level reaches a second predetermined level lower than the first predetermined level.

6. A method according to any preceding claim, wherein the flow of liquid from the fluid displacement volume (15) is controlled using a pressure maintaining valve (22) configured to maintain a pre-determined back pressure.

7. A method according to claim 6, wherein the flow of gas from the fluid displacement volume (15) is controlled using a pressure maintaining valve (22) configured to maintain a pre-determined back-pressure.

8. A method according to any preceding claim, wherein the amount of non-condensable gas is determined by:
determining a gas flow parameter relating to a gas volume or gas flow rate into the fluid displacement volume (15) over a time period based on the level signal;
determining a total flow parameter relating to the combined liquid and gas volume or combined liquid and gas flow rate into the fluid displacement volume (15) over the time period based on the liquid flow signal; and
determining a non-condensable gas parameter based on the gas flow parameter and the total flow parameter.

9. A method according to claim 8, wherein the gas flow parameter is the gas volume of non-condensable gas accumulated in the fluid displacement volume (15) over the time period calculated based on a change in the level signal and the known geometry or cross-sectional area of the fluid displacement volume; and/or
wherein the flow of liquid out of the fluid displacement volume (15) is monitored by a flow meter (24) disposed downstream of the fluid displacement volume (15), and wherein the total flow parameter is the volume of fluid flow received in the fluid displacement volume over the time period calculated based on a volume of liquid flow through the flow meter (24).

10. A method according to any preceding claim, further comprising monitoring the temperature in the fluid displacement volume (15), and wherein the amount of non-condensable gas is determined based on the liquid level signal, the liquid flow signal and the temperature in the fluid displacement volume (15).

11. A method according to any preceding claim, wherein the fluid displacement volume (15) comprises a plurality of interconnected fluid displacement chambers (16, 17), wherein the fluid flow is received in a first fluid displacement chamber (16), and wherein the level sensor (32) monitors the liquid level of a free surface in a second fluid displacement chamber (17) to generate the level signal.

12. An apparatus configured to determine the amount of non-condensable gas within a fluid flow containing both non-condensable gas and condensate liquid using the method according to any of claims 1-11, the apparatus comprising:
a fluid displacement volume (15) for receiving the fluid flow and configured so that the non-condensable gas rises and accumulates in an upper portion of the volume (15);
a level sensor (32) configured to monitor the liquid level of a free surface in the displacement volume (15) to generate a level signal;
a flow meter (24) configured to monitor the flow of liquid from the fluid displacement volume (15) to generate a liquid flow signal related to the flow rate of liquid out of the fluid displacement volume (15); and
a non-condensable gas determining module configured to determine the amount of non-condensable gas in the fluid flow based on the level signal and the liquid flow signal, wherein the determination is for a known geometry of the fluid displacement volume.

13. An apparatus according to claim 12, wherein the level sensor (32) comprises a float (34) disposed in the fluid displacement volume (15) and moveable in response to changes in the liquid level, and a position sensor (36) for monitoring the position of the float (34) to generate the level signal.

14. An apparatus according to claim 12 or 13, wherein the flow meter (24) is disposed downstream of the fluid displacement volume (15).

15. An apparatus according to any of claims 12-14, further comprising a temperature sensor (42) configured to generate a temperature signal relating to the temperature of non-condensable gas in the fluid displacement volume (15), and wherein the non-condensable gas determining module is configured to determine the amount of non-condensable gas based on the liquid level signal, the liquid flow signal and the temperature signal.

16. An apparatus according to any of claims 12 to 15, wherein the non-condensable gas determining module is configured to determine a volume of non-condensable gas accumulated in the fluid displacement volume (15) over a time period based on a change in the liquid level signal and the known geometry or cross-sectional area of the fluid displacement volume; and/or
wherein the non-condensable gas determining module is configured to calculate a volume of liquid flow received in the fluid displacement volume in the time period based on a volume of liquid flow through the flow meter (24).

17. An apparatus according to any of claims 12-16, wherein the fluid displacement volume (15) comprises a plurality of interconnected fluid displacement chambers (16, 17) including a first fluid displacement chamber (16) configured to receive the fluid flow, and a second fluid displacement chamber (17), wherein the level sensor (32) is configured to monitor the liquid level of the free surface in the second fluid displacement chamber (17) to generate the level signal.

## Patentansprüche

1. Verfahren zum Bestimmen der Menge an nicht kondensierbarem Gas in einer Fluidströmung, die sowohl nicht kondensierbares Gas als auch kondensierbare Flüssigkeit enthält, wobei das Verfahren umfasst:
Aufnehmen der Fluidströmung in einem Fluidverdrängungsvolumen (15), was bewirkt, dass das nicht kondensierbare Gas aufsteigt und sich in einem oberen Abschnitt des Volumens (15) sammelt; und
Überwachen des Flüssigkeitspegels einer freien Oberfläche in dem Fluidverdrängungsvolumen (15), um ein Pegelsignal zu erzeugen; **gekennzeichnet durch**
Überwachen der Flüssigkeitsströmung aus dem Fluidverdrängungsvolumen (15), während die Flüssigkeit aus dem Fluidverdrängungsvolumen mit derselben Rate abgelassen wird wie die Rate, mit der die Fluidströmung in das Fluidverdrängungsvolumen eintritt, und eine Gasauslassleitung des Fluidverdrängungsvolumens geschlossen ist, um ein Flüssigkeitsströmungssignal in Bezug auf die Strömungsrate von Flüssigkeit aus dem Fluidverdrängungsvolumen (15) zu generieren; und
Bestimmen der Menge an nicht kondensierbarem Gas in der Fluidströmung basierend auf dem Pegelsignal und dem Flüssigkeitsströmungssignal, wobei die Bestimmung für eine bekannte Geometrie des Fluidverdrängungsvolumens ist.

2. Verfahren nach Anspruch 1, wobei der Flüssigkeitspegel mithilfe eines Pegelsensors (32) überwacht wird, der einen in dem Fluidverdrängungsvolumen (15) angeordneten Schwimmer (34) und einen Positionssensor (36) zum Überwachen der Position des Schwimmers (34) und Generieren des Pegelsignals in Bezug auf den Flüssigkeitspegel in dem Fluidverdrängungsvolumen (15) umfasst.

3. Verfahren nach einem der vorhergehenden Ansprüche, wobei die Flüssigkeitsströmung aus dem Fluidverdrängungsvolumen (15) durch einen Strömungsmesser (24) überwacht wird, der stromabwärts des Fluidverdrängungsvolumens (15) angeordnet ist.

4. Verfahren nach einem der vorhergehenden Ansprüche, ferner umfassend Umschalten von einer Nachfüllkonfiguration zu einer Ablasskonfiguration, wenn der Flüssigkeitspegel einen ersten vorbestimmten Pegel erreicht; wobei in der Nachfüllkonfiguration eine Flüssigkeitsauslassöffnung geschlossen ist und eine Gasauslassöffnung ausgelegt ist, Gas aus dem Fluidverdrängungsvolumen (15) abzulassen; und wobei in der Ablasskonfiguration die Flüssigkeitsauslassöffnung ausgelegt ist, Flüssigkeit aus dem Fluidverdrängungsvolumen (15) abzulassen, und die Gasauslassöffnung geschlossen ist.

5. Verfahren nach Anspruch 4, ferner umfassend Umschalten von der Ablasskonfiguration zu der Nachfüllkonfiguration, wenn der Flüssigkeitspegel einen zweiten vorbestimmten Pegel erreicht, der niedriger als der erste vorbestimmte Pegel ist.

6. Verfahren nach einem der vorhergehenden Ansprüche, wobei die Flüssigkeitsströmung aus dem Fluidverdrängungsvolumen (15) mithilfe eines Druckhalteventils (22) gesteuert wird, das ausgelegt ist, einen vorbestimmten Gegendruck aufrechtzuerhalten.

7. Verfahren nach Anspruch 6, wobei die Gasströmung aus dem Fluidverdrängungsvolumen (15) mithilfe eines Druckhalteventils (22) gesteuert wird, das ausgelegt ist, einen vorbestimmten Gegendruck aufrechtzuerhalten.

8. Verfahren nach einem der vorhergehenden Ansprüche, wobei die Menge an nicht kondensierbarem Gas bestimmt wird durch:
Bestimmen eines Gasströmungsparameters in Bezug auf ein Gasvolumen oder eine Gasströmungsrate in das Fluidverdrängungsvolumen (15) über einen Zeitraum basierend auf dem Pegelsignal;
Bestimmen eines Gesamtströmungsparameters in Bezug auf das kombinierte Flüssigkeits- und Gasvolumen oder eine kombinierte Flüssigkeits- und Gasströmungsrate in das Fluidverdrängungsvolumen (15) über den Zeitraum basierend auf dem Flüssigkeitsströmungssignal; und
Bestimmen eines nicht kondensierbaren Gasparameters basierend auf dem Gasströmungsparameter und dem Gesamtströmungsparameter.

9. Verfahren nach Anspruch 8, wobei der Gasströmungsparameter das Gasvolumen von nicht kondensierbarem Gas ist, das sich in dem Fluidverdrängungsvolumen (15) über den Zeitraum angesammelt hat, berechnet basierend auf einer Änderung des Pegelsignals und der bekannten Geometrie oder Querschnittsfläche des Fluidverdrängungsvolumens; und/oder
wobei die Flüssigkeitsströmung aus dem Fluidverdrängungsvolumen (15) durch einen Strömungsmesser (24) überwacht wird, der stromabwärts des Fluidverdrängungsvolumens (15) angeordnet ist, und wobei der Gesamtströmungsparameter das Volumen einer in dem Fluidverdrängungsvolumen über den Zeitraum aufgenommenen Fluidströmung ist, berechnet basierend auf einem Volumen einer Flüssigkeitsströmung durch den Strömungsmesser (24) .

10. Verfahren nach einem der vorhergehenden Ansprüche, ferner umfassend Überwachen der Temperatur in dem Fluidverdrängungsvolumen (15), und wobei die Menge an nicht kondensierbarem Gas basierend auf dem Flüssigkeitspegelsignal, dem Flüssigkeitsströmungssignal und der Temperatur in dem Fluidverdrängungsvolumen (15) bestimmt wird.

11. Verfahren nach einem der vorhergehenden Ansprüche, wobei das Fluidverdrängungsvolumen (15) mehrere miteinander verbundene Fluidverdrängungskammern (16, 17) umfasst, wobei die Fluidströmung in einer ersten Fluidverdrängungskammer (16) aufgenommen wird, und wobei der Pegelsensor (32) den Flüssigkeitspegel einer freien Oberfläche in einer zweiten Fluidverdrängungskammer (17) überwacht, um das Pegelsignal zu generieren.

12. Einrichtung, die zum Bestimmen der Menge an nicht kondensierbarem Gas in einer Fluidströmung, die sowohl nicht kondensierbares Gas als auch kondensierbare Flüssigkeit enthält, mithilfe des Verfahrens nach einem der Ansprüche 1-11 ausgelegt ist, wobei die Einrichtung umfasst:
ein Fluidverdrängungsvolumen (15) zum Aufnehmen der Fluidströmung und derart ausgelegt, dass das nicht kondensierbare Gas aufsteigt und sich in einem oberen Abschnitt des Volumens (15) sammelt;
einen Pegelsensor (32), der ausgelegt ist, den Flüssigkeitspegel einer freien Oberfläche in dem Verdrängungsvolumen (15) zu überwachen, um ein Pegelsignal zu erzeugen;
einen Strömungsmesser (24), der ausgelegt ist, die Flüssigkeitsströmung aus dem Fluidverdrängungsvolumen (15) zu überwachen, um ein Flüssigkeitsströmungssignal in Bezug auf die Strömungsrate von Flüssigkeit aus dem Fluidverdrängungsvolumen (15) zu generieren; und
ein Modul zum Bestimmen von nicht kondensierbarem Gas, das ausgelegt ist, die Menge an nicht kondensierbarem Gas in der Fluidströmung basierend auf dem Pegelsignal und dem Flüssigkeitsströmungssignal zu bestimmen, wobei die Bestimmung für eine bekannte Geometrie des Fluidverdrängungsvolumens ist.

13. Einrichtung nach Anspruch 12, wobei der Pegelsensor (32) einen Schwimmer (34), der in dem Fluidverdrängungsvolumen (15) angeordnet und in Reaktion auf Änderungen bei dem Flüssigkeitspegel bewegbar ist, und einen Positionssensor (36) zum Überwachen der Position des Schwimmers (34) umfasst, um das Pegelsignal zu generieren.

14. Einrichtung nach Anspruch 12 oder 13, wobei der Strömungsmesser (24) stromabwärts des Fluidverdrängungsvolumens (15) angeordnet ist.

15. Einrichtung nach einem der Ansprüche 12-14, ferner umfassend einen Temperatursensor (42), der ausgelegt ist, ein Temperatursignal in Bezug auf die Temperatur von nicht kondensierbarem Gas in dem Fluidverdrängungsvolumen (15) zu erzeugen, und wobei das Modul zum Bestimmen von nicht kondensierbarem Gas ausgelegt ist, die Menge an nicht kondensierbarem Gas basierend auf dem Flüssigkeitspegelsignal, dem Flüssigkeitsströmungssignal und dem Temperatursignal zu bestimmen.

16. Einrichtung nach einem der Ansprüche 12 bis 15, wobei das Modul zum Bestimmen von nicht kondensierbarem Gas ausgelegt ist, ein Volumen an nicht kondensierbarem Gas, das sich in dem Fluidverdrängungsvolumen (15) über einen Zeitraum angesammelt hat, basierend auf einer Änderung des Flüssigkeitspegelsignals und der bekannten Geometrie oder Querschnittsfläche des Fluidverdrängungsvolumens zu bestimmen; und/oder
wobei das Modul zum Bestimmen von nicht kondensierbarem Gas ausgelegt ist, ein Volumen einer in dem Fluidverdrängungsvolumen in dem Zeitraum aufgenommenen Flüssigkeitsströmung basierend auf einem Volumen einer Flüssigkeitsströmung durch den Strömungsmesser (24) zu berechnen.

17. Einrichtung nach einem der Ansprüche 12-16, wobei das Fluidverdrängungsvolumen (15) mehrere miteinander verbundene Fluidverdrängungskammern (16, 17) einschließlich einer ersten Fluidverdrängungskammer (16), die ausgelegt ist, die Fluidströmung aufzunehmen, und einer zweiten Fluidverdrängungskammer (17) umfasst, wobei der Pegelsensor (32) ausgelegt ist, den Flüssigkeitspegel der freien Oberfläche in der zweiten Fluidverdrängungskammer (17) zu überwachen, um das Pegelsignal zu generieren.

## Revendications

1. Procédé de détermination de la quantité de gaz non condensable dans un écoulement de fluide contenant à la fois du gaz non condensable et du liquide condensé, le procédé comprenant :
la réception de l'écoulement de fluide dans un volume de déplacement de fluide (15) amenant le gaz non condensable à monter et s'accumuler dans une partie supérieure du volume (15) ; et
la surveillance du niveau de liquide d'une surface libre dans le volume de déplacement de fluide (15) pour générer un signal de niveau ; **caractérisé par**
la surveillance de l'écoulement de liquide hors du volume de déplacement de fluide (15) pendant que le liquide est évacué du volume de déplacement de fluide au même débit que le débit de l'écoulement de fluide entrant dans le volume de déplacement de fluide et qu'une conduite de sortie de gaz du volume de déplacement de fluide est fermée, pour générer un signal d'écoulement de liquide relatif au débit de liquide hors du volume de déplacement de fluide (15) ; et
la détermination de la quantité de gaz non condensable dans l'écoulement de fluide sur la base du signal de niveau et du signal d'écoulement de liquide, la détermination étant pour une géométrie connue du volume de déplacement de fluide.

2. Procédé selon la revendication 1, le niveau de liquide étant surveillé en utilisant un capteur de niveau (32) comprenant un flotteur (34) disposé dans le volume de déplacement de fluide (15) et un capteur de position (36) pour surveiller la position du flotteur (34) et générer le signal de niveau relatif au niveau de liquide dans le volume de déplacement de fluide (15).

3. Procédé selon l'une quelconque des revendications précédentes, l'écoulement de liquide hors du volume de déplacement de fluide (15) étant surveillé par un débitmètre (24) disposé en aval du volume de déplacement de fluide (15).

4. Procédé selon l'une quelconque des revendications précédentes, comprenant en outre le passage d'une configuration de remplissage à une configuration d'évacuation lorsque le niveau de liquide atteint un premier niveau prédéterminé ; dans la configuration de remplissage, un orifice de sortie de liquide étant fermé et un orifice de sortie de gaz étant configuré pour évacuer le gaz du volume de déplacement de fluide (15) ; et, dans la configuration d'évacuation, l'orifice de sortie de liquide étant configuré pour évacuer le liquide du volume de déplacement de fluide (15) et l'orifice de sortie de gaz étant fermé.

5. Procédé selon la revendication 4, comprenant en outre le passage de la configuration d'évacuation à la configuration de remplissage lorsque le niveau de liquide atteint un second niveau prédéterminé inférieur au premier niveau prédéterminé.

6. Procédé selon l'une quelconque des revendications précédentes, l'écoulement de liquide à partir du volume de déplacement de fluide (15) étant contrôlé en utilisant une soupape de maintien de pression (22) configurée pour maintenir une contre-pression prédéterminée.

7. Procédé selon la revendication 6, l'écoulement de gaz provenant du volume de déplacement de fluide (15) étant contrôlé en utilisant une soupape de maintien de pression (22) configurée pour maintenir une contre-pression prédéterminée.

8. Procédé selon l'une quelconque des revendications précédentes, la quantité de gaz non condensable étant déterminée par :
la détermination d'un paramètre d'écoulement de gaz relatif à un volume de gaz ou à un débit de gaz dans le volume de déplacement de fluide (15) sur une période de temps basée sur le signal de niveau ;
la détermination d'un paramètre d'écoulement total relatif au volume combiné de liquide et de gaz ou au débit combiné de liquide et de gaz dans le volume de déplacement de fluide (15) sur la période de temps basée sur le signal d'écoulement de liquide ; et
la détermination d'un paramètre de gaz non condensable sur la base du paramètre d'écoulement de gaz et du paramètre d'écoulement total.

9. Procédé selon la revendication 8, le paramètre d'écoulement de gaz étant le volume de gaz de gaz non condensable accumulé dans le volume de déplacement de fluide (15) sur la période de temps calculée sur la base d'un changement du signal de niveau et de la géométrie connue ou de la surface de section transversale du volume de déplacement de fluide ; et/ou
l'écoulement de liquide hors du volume de déplacement de fluide (15) étant surveillé par un débitmètre (24) disposé en aval du volume de déplacement de fluide (15), et le paramètre d'écoulement total étant le volume d'écoulement de fluide reçu dans le volume de déplacement de fluide sur la période de temps calculée sur la base d'un volume d'écoulement de liquide à travers le débitmètre (24).

10. Procédé selon l'une quelconque des revendications précédentes, comprenant en outre la surveillance de la température dans le volume de déplacement de fluide (15), et la quantité de gaz non condensable étant déterminée sur la base du signal de niveau de liquide, du signal d'écoulement de liquide et de la température dans le volume de déplacement de fluide (15).

11. Procédé selon l'une quelconque des revendications précédentes, le volume de déplacement de fluide (15) comprenant une pluralité de chambres de déplacement de fluide interconnectées (16, 17), l'écoulement de fluide étant reçu dans une première chambre de déplacement de fluide (16), et le capteur de niveau (32) surveillant le niveau de liquide d'une surface libre dans une seconde chambre de déplacement de fluide (17) pour générer le signal de niveau.

12. Appareil configuré pour déterminer la quantité de gaz non condensable dans un écoulement de fluide contenant à la fois du gaz non condensable et du liquide condensé en utilisant le procédé selon l'une quelconque des revendications 1 à 11, l'appareil comprenant :
un volume de déplacement de fluide (15) pour recevoir l'écoulement de fluide et configuré de sorte que le gaz non condensable monte et s'accumule dans une partie supérieure du volume (15) ;
un capteur de niveau (32) configuré pour surveiller le niveau de liquide d'une surface libre dans le volume de déplacement (15) pour générer un signal de niveau ;
un débitmètre (24) configuré pour surveiller l'écoulement de liquide du volume de déplacement de fluide (15) pour générer un signal d'écoulement de liquide relatif au débit de liquide hors du volume de déplacement de fluide (15) ; et
un module de détermination de gaz non condensable configuré pour déterminer la quantité de gaz non condensable dans l'écoulement de fluide sur la base du signal de niveau et du signal d'écoulement de liquide, la détermination étant pour une géométrie connue du volume de déplacement de fluide.

13. Appareil selon la revendication 12, le capteur de niveau (32) comprenant un flotteur (34) disposé dans le volume de déplacement de fluide (15) et déplaçable en réponse à des changements du niveau de liquide, et un capteur de position (36) pour surveiller la position du flotteur (34) afin de générer le signal de niveau.

14. Appareil selon la revendication 12 ou 13, le débitmètre (24) étant disposé en aval du volume de déplacement de fluide (15).

15. Appareil selon l'une quelconque des revendications 12 à 14, comprenant en outre un capteur de température (42) configuré pour générer un signal de température relatif à la température du gaz non condensable dans le volume de déplacement de fluide (15), et le module de détermination du gaz non condensable étant configuré pour déterminer la quantité de gaz non condensable sur la base du signal de niveau de liquide, du signal d'écoulement de liquide et du signal de température.

16. Appareil selon l'une quelconque des revendications 12 à 15, le module de détermination de gaz non condensable étant configuré pour déterminer un volume de gaz non condensable accumulé dans le volume de déplacement de fluide (15) sur une période de temps sur la base d'un changement du signal de niveau de liquide et la géométrie connue ou la surface de section transversale du volume de déplacement de fluide ; et/ou
le module de détermination de gaz non condensable étant configuré pour calculer un volume d'écoulement de liquide reçu dans le volume de déplacement de fluide dans la période de temps sur la base d'un volume d'écoulement de liquide à travers le débitmètre (24).

17. Appareil selon l'une quelconque des revendications 12 à 16, le volume de déplacement de fluide (15) comprenant une pluralité de chambres de déplacement de fluide interconnectées (16, 17) comprenant une première chambre de déplacement de fluide (16) configurée pour recevoir l'écoulement de fluide et une seconde chambre de déplacement de fluide (17), le capteur de niveau (32) étant configuré pour surveiller le niveau de liquide de la surface libre dans la seconde chambre de déplacement de fluide (17) pour générer le signal de niveau.
